Europäisches Patentamt

European Patent Office

Office européen des brevets

(19)

(11) Publication number: **0 246 504 B1**

# EUROPEAN PATENT SPECIFICATION

(12)

(45) Date of publication of patent specification: **26.08.92**

(51) Int. Cl.⁵: **C07C 45/41**, C07C 29/136, C07C 33/32, C07C 47/21, C07C 47/232

(21) Application number: **87106652.8**

(22) Date of filing: **07.05.87**

(54) **Process for the preparation of a mixture of an aldehyde with the corresponding alcohol.**

(30) Priority: **12.05.86 JP 108274/86**

(43) Date of publication of application:
**25.11.87 Bulletin 87/48**

(45) Publication of the grant of the patent:
**26.08.92 Bulletin 92/35**

(84) Designated Contracting States:
**CH DE IT LI**

(56) References cited:
**GB-A- 2 013 202**

**CHEMICAL ABSTRACTS, vol. 100, no. 7, 13th February 1984, page 642, column 1, abstract no. 68730b, Columbus, Ohio, US; DAIICHI SEIYAKU CO., LTD. "Tyrosinol derivatives", & JP - A - 58 33866**

**HOUBEN-WEYL "Methoden der organischen Chemie", vol. IV/1d, 1981, Georg Thieme Verlag, Stuttgart;**

(73) Proprietor: **Shin-Etsu Chemical Co., Ltd.**
**6-1, Otemachi 2-chome**
**Chiyoda-ku Tokyo 100(JP)**

(72) Inventor: **Takehiko, Fukumoto**
**Kirinoki Ryo 27-20, Sanai-cho**
**Joetsu-shi Niigata-ken(JP)**
Inventor: **Akira, Yamamoto**
**2-14-45, Gochi**
**Joetsu-shi Niigata-ken(JP)**

(74) Representative: **Blum, Rudolf Emil Ernst et al**
**c/o E. Blum & Co Patentanwälte Vorderberg 11**
**CH-8044 Zürich(CH)**

EP 0 246 504 B1

## Description

The present invention concerns a process for the preparation of a mixture of an aldehyde with the corresponding alcohol by reducing the corresponding carboxylic acid. According to said process the carboxylic acid is first reacted with a chloroformate ester and said intermediate product thereafter reduced with an alkali metal borohydride to form the aldehyde of the carboxylic acid used as starting material mixed with the corresponding alcohol.

## DESCRIPTION OF THE PRIOR ART

When a carboxylic acid is submitted to a reduction reaction in order to prepare the corresponding aldehyde or the corresponding alcohol, according to the prior art usually the corresponding carboxylic acid was reduced dissolved in diethyl ether or tetrahydrofuran using lithium aluminum hydride $LiAlH_4$ as the reducing agent. The applicability of this reducing method, however, is limited even by setting aside the problem of expensiveness of this reagent to prohibit industrial application of the method. For example, reduction of an unsaturated carboxylic acid by this method is not always suitable for the preparation of the corresponding unsaturated aldehyde or alcohol since the reduction takes place not selectively at the carboxyl group alone but also the unsaturated linkage in the unsaturated carboxylic acid is also susceptible to reduction. When cinnamic acid is reduced in an ether solution by using lithium aluminum hydride as the reducing agent, for example, the reaction product is not the desired cinnamyl alcohol but dihydro-cinnamyl alcohol as is taught by R.F. Nystron, et al. in Journal of the American Chemical Society, volume 69, page 2548 (1947).

The unsaturated linkage in the unsaturated carboxylic acid may be protected from reduction by using a reducing agent system of lithium aluminum hydride combined with anhydrous aluminum chloride. For example, sorbic acid $CH_3CH=CH-CH=CH-COOH$ can be reduced by this method into hexa-2,4-dienol $CH_3CH=CH-CH=CH-CH_2OH$. This method, however, is not practical due to the extremely low yield of the reaction product of, for example, only 20% in the above mentioned reduction of sorbic acid. Moreover, the reaction product in the reduction of a carboxylic acid using lithium aluminum hydride is always the corresponding alcohol while the corresponding aldehyde compound, which should have been formed at the intermediate stage, can hardly be obtained as the final product.

Sodium borohydride is less expensive as a reducing agent than lithium aluminum hydride and is used in various synthetic reactions. The reducing power of this reducing agent is, however, not strong enough to reduce a carboxylic acid into a corresponding aldehyde or alcohol. The reducing power of sodium borohydride can be strengthened by performing the reducing reaction in diglyme as the solvent by combining sodium borohydride with anhydrous aluminum chloride as is taught by H.C. Brown, et al. in Journal of the American Chemical Society, volume 75, page 6263 (1953) or by combining sodium borohydride with boron trifluoride $BF_3$ as is taught by G.R. Pettit, et al. in Journal of Organic Chemistry, volume 27, page 2127 (1962).

These improved methods for increasing the reducing power of sodium borohydride are still not quite satisfactory due to the use of a Lewis acid such as anhydrous aluminum chloride and boron trifluoride. Namely, the procedure of the reaction is usually troublesome and the reaction is sometimes accompanied by undesirable side reactions when the product is not sufficiently stable resulting in decrease in the yield of the desired product. The selectivity of the reducing reaction is also not without problems. In addition, the diglyme used as the solvent can be recovered with some difficulties and the waste material containing aluminum chloride may be responsible for very serious environmental pollution when the waste water is discharged to public waterway without appropriate treatment of sewage disposal.

Lithium aluminum hydride and sodium borohydride both belong to a class of typical complex compounds capable of reducing various kinds of organic compounds by releasing hydrogen in the form of hydride ions. The problems in these hydrides as an industrial reducing agent are the expensiveness of lithium aluminum hydride having a relatively high reducing power and the relatively low reducing power of the less expensive sodium borohydride necessitating combined use of an auxiliary reagent to cause complicacy of the process and low yield of the product. At any rate, each of these hydrides is not suitable as a reducing agent when a carboxylic acid should be reduced to the corresponding aldehyde and not to the alcohol.

In Houben-Weyl's "Methoden der Organischen Chemie", vol. IV/ld, pp. 126-128, there is disclosed a process for the preparation of alcohols starting from the corresponding carboxylic acid, which process comprises two reaction steps:

(a) the carboxylic acid is reacted with a chloroformic acid ester in the presence of triethylamine in absolute tetrahydrofurane to yield an intermediate acylalkylcarbonate and thereafter

(b) said intermediate acylalkylcarbonate is reduced with 2,5 moles of sodium borohydride per mole of carboxylic acid in aqueous tetrahydrofurane to yield the corresponding alcohol. (see page 127)

Specifically there is described on page 127, line 5 from the bottom, that said twostep reaction is also applicable for preparing cinnamic alcohol starting from cinnamic acid.

If, however, an aromatic carboxylic acid having a hydroxy group in the ortho-position to the group COOH is submitted to said two step reaction, then in step (a) the COOH group is converted into the corresponding acid anhydride by the reaction with the chloroformic acid alkyl ester and furthermore the hydroxy group esterified by the chloroformic acid ester. In step (b) the sodium borohydride, however, reduces the acid anhydride group to the corresponding methyl group, yielding as final product a phenyl nucleus having a methyl substituent and in ortho-position to it a hydroxy group. With regrad to this we refer to Houbel-Weyl, page 128, lines 1 to 18.

In Chemical Abstracts, vol. 100, no. 7, 13th February 1984, page 642, column 1, abstract no. 68730b, there is described a process for preparing tyrosinol derivatives by performing a N-acylation of the free $NH_2$ group of the tyrosine and by submitting said acylated product to a reduction with sodium borohydride in tetrahydrofurane, through which reduction the COOH group of the N-acylated tyrosine is converted to a corresponding primary alcohol, i.e. into a group of formula $-CH_2OH$.

Accordingly, from the above stated citations of Houbel-Weyl and Chemical Abstracts, there can be seen that the free COOH group or a mixed anhydride thereof with a monoester of the carbonic acid can be reduced with sodium borohydride to yield the corresponding primary alcohol or even a methyl group, provided that the carboxylic acid group of the starting material is bonded to a phenyl nucleus. There cannot be taken from said publications a reference that it is also possible to reduce the carboxylic acid group of the corresponding intermediate product or a corresponding free carboxylic acid to the aldehyde group using sodium borohydride as reducing agent.

In the British patent application 2 013 202 A there is described a process for the preparation of aromatic aldehydes by first converting the corresponding aromatic acid with an alkyl chlorocarbonate into the mixed anhydride of the aromatic acid with the carbonic acid monoalkyl ester and by hydrogenating said mixed anhydride in the presence of a hydrogenation catalyst to yield the corresponding aldehyde.

It was the aim of the present invention to provide a process for the preparation of a mixture of an aldehyde with the corresponding alcohol by reducing the corresponding carboxylic acid. Said process should be applicable also to carboxylic acids which have in their structure alkenyl groups or alkynyl groups and, during which reduction process said aliphatically unsaturated groups remain unaltered.

It was found out that said aims can be achieved by performing a two-step reaction in which first a mixed anhydride with a monoester of the carbonic acid is formed and said mixed anhydride reduced using an alkali-metal borohydride in an amount of 1,0-2,0 moles of alkali-metal borohydride per mole of the starting material.

## DESCRIPTION OF THE INVENTION

One object of the present invention is a process for the preparation of a mixture of an aldehyde having the general formula IV

RCHO     IV

with a corresponding alcohol of formula V

$RCH_2OH$     V

in which formulae

R is a monovalent hydrocarbon group having 1 - 20 carbon atoms which hydrocarbon group is selected from the class comprising alkyl groups, alkenyl groups, alkynyl groups, aryl groups and aryl substituted alkenyl groups,

by reducing a corresponding carboxylic acid of formula I

RCOOH     I

in which

R is as defined above, which process is characterized in that it comprises the following reaction steps:

(a) reacting the carboxylic acid of formula I with a chloroformate ester of formula II

ClCO-OR$^1$      II

in which

R$^1$ is a monovalent hydrocarbon group having 1 - 20 carbon atoms, which is selected from the group which comprises alkyl, alkenyl, alkynyl and aryl groups,

in the presence of an acid acceptor to form an intermediate compound of formula III

R-CO-O-CO-OR$^1$,      III

in which

R and R$^1$ are as defined above and

(b) reducing the intermediate compound of formula III with 1,0 to 2,0 moles of an alkali metal borohydride, per mole of the acid of formula I to form the mixture of the aldehyde of formula IV with the alcohol of formula V.

According to the inventive process the carboxylic acid used as starting material is reduced with the metal borohydride, not in its free form but as an acid anhydride with the corresponding monoester of the carbonic acid. This method of reduction is very advantageous because, if not to mention the inexpensiveness of the reducing agent, the reducing reaction can be controlled to give an aldehyde compound which is intermediate in the course of the full reduction of the acid into an alcohol or a mixture of the aldehyde with the corresponding alcohol.

If, furthermore, the used starting material is an unsaturated acid, the unsaturated linkage is not affected as a result of the selective reduction at the carboxyl group of the acid alone. The inventive process, accordingly, is applicable to the reduction of carboxylic acids having the above stated formula I, wherein R is a monovalent hydrocarbon atom having 1- 20 carbon atoms selected from the above stated group. It, however, is specially preferred for reducing such carboxylic acids, in which R is an unsaturated group yielding the corresponding unsaturated aldehyde or a mixture of the unsaturated aldehyde with the unsaturated alcohol.

The chloroformate ester of formula II which is reacted in the reaction step (a) with a corresponding carboxylic acid, is preferably a corresponding alkyl chloroformate in which R$^1$ is an alkyl group having 1 - 20 carbon atoms. Specially preferred is methyl chloroformate and ethyl chloroformate because of its inexpensiveness.

The reaction step (a), i.e. the reaction of the carboxylic acid of formula I with the chloroformate ester of formula II is performed in the presence of an acid acceptor and preferably in an organic solvent at a temperature in the range from -20°C to +20°C. Preferred acid acceptors are organic bases, like tertiary amines, for example triethyl amine and pyridine. The organic solvent suitable as the reaction medium is preferably an ether, like e.g. a dialkyl ether, for instance diethyl-ether, or a cyclic ether, like e.g. tetrahydrofuran, 1,2-dimethoxy-ethane, i.e. diglyme, and the like and should desirably be completely dehydrated prior to use. The intermediate compound is readily formed by adding the chloroformate ester into a solution of the starting carboxylic acid kept at the above mentioned temperature by the dehydrochlorination reaction between the reactant. The hydrogen chloride formed by the reaction is caught by the acid acceptor to form precipitates such as triethyl amine hydrochloride. Although the reaction mixture containing the precipitates of the salt can be used as such in the next step of reduction, it is preferable that the precipitates should be removed by filtration and the filtrate containing the intermediate compound is used in the step of reduction in order to improve the yield of the product. The intermediate compound of the general formula (III) is generally an unstable compound so that the reaction mixture containing the interme-iate compound should be processed as quickly as possible in order to minimize the possible loss thereof by decomposition.

The reaction of reduction is performed by adding the above obtained solution containing the intermediate compound dropwise into a suspension of the metal borohydride in an organic solvent. The reaction temperature should be in the range from -50 °C to +30 °C. When an aldehyde compound is the desired product, the temperature should preferably be in the range from -50°C to 0°C so that the yield of the aldehyde compound can be increased relative to the alcohol compound. The solvent used in this step may be the same one as in the reaction of the starting carboxylic acid and the chloroformate ester including dialkyl ethers, e.g. diethyl ether, cyclic ethers, e.g., tetrahydrofuran, 1,2-dimethoxy ethane, i.e. diglyme, and the like. It is not necessary that the solvent used in the reaction of reduction is anhydrous and a mixture of water and an organic solvent can be used without particularly disadvantageous effect provided that the reactants are soluble in the medium. Tetrahydrofuran is one of the preferred organic solvents which can be used in both steps for the preparation of the intermediate compound and for the reduction of the same.

The amount of the metal borohydride used in the reduction depends on the desired reaction product i.e. the mixture which may be either rich in the aldehyde compound or rich in the alcohol compound. When the desired product is a mixture with a high aldehyde content, the metal borohydride should be used in an amount in the range from 1.0 to 1.5 moles per mole of the starting carboxylic acid and the reaction should be continued for 2 to 6 hours. If in the mixture of the aldehyde compound and the alcohol compound. an higher alcohol content is desired, on the other hand, the amount of the reducing agent should be increased to 1.5 to 2,0 moles per mole of the starting carboxylic acid and the reaction should be continued for 1 to 5 hours. The yield of the reaction products is usually lower under the reaction conditions where the major product is the aldehyde compound than under the reaction conditions where the major product is the alcohol compound. When the amount of the metal borohydride is increased to 3 moles per mole of the starting carboxylic acid, the reaction of reduction would be complete so that the resultant reaction product can be substantially free from the aldehyde compound as the intermediate reduction product. After completion of the reaction of reduction, the reaction mixture was admixed with hydrochloric acid of a concentration of, for example, 10 to 20% followed by phase separation and the desired product of the alcohol or aldehyde compound can readily be isolated from the organic phase by a conventional procedure.

When the inventive method was applied to the reduction of 1 mole of sorbic acid $CH_3CH=CHCH=CHCOOH$ which is first reacted with ethyl chloroformate $ClCOOC_2H_5$ to give an intermediate compound followed by reduction of the same with 2.0 moles of sodium borohydride in tetrahydrofuran at 10 to 15 °C, the resultant reaction mixture contained 9% by weight of sorbic aldehyde $CH_3CH=CHCH=CHCHO$ and 58% by weight of sorbyl alcohol $CH_3CH=CHCH=CH_2OH$. When 1 mole of cinnamic acid $C_6H_5CH=CHCOOH$ was first reacted with methyl chloroformate $ClCOOCH_3$ to give an intermediate compound followed by reduction of the same with 1.3 moles of sodium borohydride in tetrahydrofuran at -10 to 5 °C, the resultant reaction mixture contained 25% by weight of cinnamic aldehyde $C_6H_5CH=CHCHO$ and 20% by weight of cinnamyl alcohol $C_6H_5CH=CHCH_2OH$.

In the following, examples and comparative examples are given to illustrate the method of the invention in more detail but not to limit the scope of the invention in any way.

Example 1.

Into a solution prepared by dissolving 28 g (0.25 mole) of sorbic acid in 100 ml of tetrahydrofuran with admixture of 25 g of triethyl amine kept at 0 to 10 °C were added dropwise 27 g of ethyl chloroformate over a period of 30 minutes with agitation. After completion of the dropwise addition of ethyl chloroformate, the mixture was further agitated for additional 30 minutes at 10 °C and then quickly filtered to remove the precipitates of triethyl amine hydrochloride. Thereafter, a suspension of 19 g (0.5 mole) of sodium borohydride in 100 ml of tetrahydrofuran was added dropwise into the above obtained filtrate solution kept at 10 to 15 °C under an atmosphere of nitrogen. After completion of the dropwise addition of the suspension, the reaction mixture kept at 20 °C was further agitated for additional 30 hours and 100 ml of a 10% hydrochloric acid were added thereto followed by phase separation. The organic solution taken by phase separation was subjected to evaporation in a rotary evaporator to remove tetrahydrofuran and then distilled under reduced pressure to give 16.4 g of a fraction boiling at 80 to 86 °C under a pressure of 1333 Pa (10 mmHg) as a product. Gas chromatographic analysis of this product indicated that the product was a 14:86 by weight mixture of 2,4-hexadien-1-al, i.e. sorbic aldehyde, and 2,4-hexadien-1-ol, i.e. sorbyl alcohol. The yield was 67% of the theoretical value.

Comparative Example 1a

Into a solution of 12 g (0.31 mole) of lithium aluminum hydride in 100 ml of anhydrous tetrahydrofuran kept at 0 to 10 °C were added gradually 13 g (0.1 mole) of anhydrous aluminum chloride and the mixture was agitated for 30 minutes at 10 °C. In the next place, a solution of 28 g (0.25 mole) of sorbic acid dissolved in 100 ml of anhydrous tetrahydrofuran was added dropwise to the reaction mixture kept at 0 to 20 °C over a period of 1 hour. After completion of the dropwise addition of the solution, the reaction mixture was admixed with 100 ml of a 10% hydrochloric acid followed by phase separation. The organic solution taken by phase separation was subjected to evaporation of tetrahydrofuran in a rotary evaporator and then distilled under reduced pressure to give 4.9 g of 2,4-hexadien-1-ol in a yield of 20% of the theoretical value.

Comparative Example 1b.

Into a solution of 12 g (0.3 mole) of sodium borohydride in 100 ml of diglyme were added 13 g (0.1 mole) of anhydrous aluminum chloride gradually at 0 to 10 °C and the mixture was agitated for 30 minutes at 10 °C. Thereafter, a solution of 28 g (0.25 mole) of sorbic acid in 100 ml of anhydrous diglyme was added dropwise to the mixture at 0 to 20 °C over a period of 1 hour. After completion of the dropwise addition of the solution, the mixture was admixed with 100 ml of a 10% hydrochloric acid followed by phase separation and the organic solution taken by phase separation was distilled under reduced pressure to give 4.0 g of a fraction boiling at 79 to 81 °C under a pressure of 2000 Pa (15 mmHg). Gas chromatographic analysis of this product indicated that the product was composed of 5.1% by weight of 2,4-hexadien-1-ol and 94.9% by weight of n-hexyl alcohol. The yields of these products were 5.1% and 19%, respectively, of the theoretical values relative to the starting amount of the sorbic acid.

Example 2.

The experimental conditions in each of the four synthetic experiments were approximately the same as in Example 1 except that the starting carboxylic acid was cinnamic acid in an amount of 37 g (0.25 mole) instead of 28 g (0.25 mole) of sorbic acid and the reaction temperature and the amount of sodium borohydride were varied as shown in the table below, which also includes the yield of the products relative to the theoretical value and the weight ratio of cinnamic aldehyde to the cinnamyl alcohol in each of the experiments.

| Reaction temperature, °C | Moles of NaBH$_4$ per mole of acid | Yield, % | Ratio of aldehyde to alcohol |
|---|---|---|---|
| 10 - 15 | 2.0 | 69 | 12:88 |
| 0 - 10 | 1.5 | 55 | 31:69 |
| 10 - 5 | 1.2 | 45 | 55:45 |

Comparative Example 2.

Into a solution of 12 g (0.31 mole) of lithium aluminum hydride in 100 ml of anhydrous diethyl ether was added dropwise a solution prepared by dissolving 37 g (0.25 mole) of cinnamic acid in 100 ml of anhydrous diethyl ether at a temperature of 0 to 20 °C. After completion of the dropwise addition of the solution, the mixture was further agitated for additional 30 minutes at 20 °C and then admixed with 100 ml of a 10% hydrochloric acid followed by phase separation. The organic solution taken by phase separation was subjected to evaporation of the ether and then distilled under reduced pressure to give 11 g of dihydrocinnamyl alcohol, which yield was 31% of the theoretical value, but neither cinnamyl alcohol nor cinnamic aldehyde could be obtained.

**Claims**

**1.** Process for the preparation of a mixture of an aldehyde having the general formula IV

RCHO     IV

with a corresponding alcohol of formula V

RCH$_2$OH     V

in which formulae
R is a monovalent hydrocarbon group having 1 - 20 carbon atoms which hydrocarbon group is selected from the class comprising alkyl groups, alkenyl groups, alkynyl groups,aryl groups and aryl substituted alkenyl groups,
by reducing a corresponding carboxylic acid of formula I

RCOOH     I

in which

R is as defined above, characterized in that the process comprises the following reaction steps:
(a) reacting the carboxylic acid of formula I with a chloroformate ester of formula II

ClCO-OR$^1$    II

in which

R$^1$ is a monovalent hydrocarbon group having 1 - 20 carbon atoms, which is selected from the group which comprises alkyl, alkenyl, alkynyl and aryl groups,
in the presence of an acid acceptor to form an intermediate compound of formula III

R-CO-O-CO-OR$^1$,    III

in which

R and R$^1$ are as defined above and
(b) reducing the intermediate compound of formula III with 1,0 - 2,0 moles of an alkali metal borohydride per mole of acid of formula I to form the mixture of the aldehyde of formula IV with the alcohol of formula V.

2.    Process according to claim 1, characterized in that in the step (b) there is used as reducing agent the lithium borohydride or preferably the sodium borohydride.

3.    Process according to claim 1 or 2, characterized in that in step (a) there is used as chloroformate ester of formula II a corresponding ester wherein R$^1$ is alkyl, preferably methyl or ethyl.

4.    Process according to one of the claims 1 through 3, characterized in that a mixture of an aldehyde of formula IV with an alcohol of formula V is prepared in which R is an alkenyl group having 1 - 20 carbon atoms or an aryl substituted alkenyl group.

5.    Process according to one of the claims 1 through 4, characterized in that the metal borohydride is used in the reaction step (b) in an amount of 1,0 to 1,5 mol per mol of the carboxylic acid of formula I used in step (a).

6.    Process according to one of the claims 1 - 5, characterized in that the reduction reaction (b) is performed at a temperature in the range of -50º C to +30º C, preferably in the range of -50º C to 0º C.

7.    Process according to one of the claims 4 through 6 wherein the carboxylic acid of formula I is sorbic acid or cinnamic acid.

**Patentansprüche**

1.    Verfahren zur Herstellung einer Mischung aus einem Aldehyd der allgemeinen Formel IV

RCHO    IV

und einem entsprechenden Alkohol der Formel V

RCH$_2$OH    V ,

wobei in den Formeln
R eine einwertige Kohlenwasserstoffgruppe mit 1 - 20 Kohlenstoffatomen ist, und die Kohlenwasserstoffgruppe aus der Gruppe ausgewählt ist, welche Alkylgruppen, Alkenylgruppen, Alkinylgruppen, Arylgruppen und arylsubstituierte Alkenylgruppen umfasst,
indem man eine entsprechende Carbonsäure der Formel I

RCOOH    I

reduziert, in welcher

R wie oben definiert ist,

dadurch gekennzeichnet, dass das Verfahren die folgenden Reaktionsschritte umfasst:

(a) Umsetzung der Carbonsäure der Formel I mit einem Chlorameisensäureester der Formel II

$ClCO-OR^1$    II ,

in welchem

$R^1$ eine einwertige Kohlenwasserstoffgruppe mit 1 - 20 Kohlenstoffatomen ist, die aus der Gruppe ausgewählt ist, welche Alkyl-, Alkenyl-, Alkinyl- und Arylgruppen umfasst,

in Anwesenheit eines Säureakzeptors unter Ausbildung eines Zwischenproduktes der Formel III

$R-CO-O-CO-OR^1$    III ,

in welchem

R und $R^1$ wie oben definiert sind und

(b) Reduktion des Zwischenproduktes der Formel III mit 1,0 - 2,0 Molen eines Alkalimetallborhydrides pro Mol der Säure der Formel I unter Ausbildung der Mischung des Aldehydes der Formel IV mit dem Alkohol der Formel V.

2. Verfahren nach Anspruch 1, dadurch gekennzeichnet, dass man in der Stufe (b) als Reduktionsmittel das Lithiumborhydrid oder vorzugsweise das Natriumborhydrid verwendet.

3. Verfahren nach Anspruch 1 oder 2, dadurch gekennzeichnet, dass man in der Stufe (a) als Chlorameisensäureester der Formel II einen entsprechenden Ester verwendet, in welchem

$R^1$ Alkyl, vorzugsweise Methyl oder Aethyl ist.

4. Verfahren nach einem der Ansprüche 1 bis 3, dadurch gekennzeichnet, dass eine Mischung aus einem Aldehyd der Formel IV mit einem Alkohol der Formel V hergestellt wird, in welcher R eine Alkenylgruppe mit 1 - 20 Kohlenstoffatomen oder eine arylsubstituierte Alkenylgruppe ist.

5. Verfahren nach einem der Ansprüche 1 bis 4, dadurch gekennzeichnet, dass das Metallborhydrid in dem Reaktionsschritt (b) in einer Menge von 1,0 bis 1,5 Mol pro Mol der im Schritt (a) eingesetzten Carbonsäure der Formel I verwendet wird.

6. Verfahren nach einem der Ansprüche 1 bis 5, dadurch gekennzeichnet, dass die Reduktionsreaktion (b) bei einer Temperatur im Bereich von -50° C bis +30° C, vorzugsweise im Bereich von -50° C bis 0° C durchgeführt wird.

7. Verfahren gemäss einem der Ansprüche 4 bis 6, dadurch gekennzeichnet, dass die Carbonsäure der Formel I Sorbinsäure oder Zimtsäure ist.

**Revendications**

1. Procédé pour la préparation d'un mélange d'un aldéhyde ayant la formule générale IV

RCHO    IV

avec un alcool correspondant de formule V

$RCH_2OH$    V

formules dans lesquelles,

R est un groupe hydrocarboné monovalent ayant de 1 - 20 atomes de carbone, lequel groupe hydrocarboné est choisi dans la classe comprenant les groupes alkyle, les groupes alcényle, les groupes alkynyle, les groupes aryle et les groupes alcényle substitués par aryle,

en réduisant un acide carboxylique correspondant de formule I

8

RCOOH     I

dans laquelle

    R est tel que défini ci-dessus, caractérisé en ce que le procédé comprend les étapes réactionnelles suivantes:

    (a) mise en réaction de l'acide carboxylique de formule I avec un ester de chloroformate de formule II

$ClCO-OR^1$     II

dans laquelle

    $R^1$ est un groupe hydrocarboné monovalent ayant 1 - 20 atomes de carbone, lequel est choisi dans le groupe qui comprend des groupes alkyle, alcényle, alkynyle et aryle,

    en présence d'un accepteur d'acide pour former un composé intermédiaire de formule III

$R-CO-O-CO-OR^1$,     III

dans laquelle

    R et $R^1$ sont tels que définis ci-dessus et

    (b) réduction du composé intermédiaire de formule III avec 1,0 - 2,0 moles d'un hydrure de bore des métaux alcalins par mole d'acide de formule I pour former le mélange de l'aldéhyde de formule IV avec l'alcool de formule V.

**2.** Procédé selon la revendication 1, caractérisé en ce qu'au cours de l'étape (b) on utilise l'hydrure de bore de lithium ou de préférence l'hydrure de bore de sodium en tant qu'agent de réduction.

**3.** Procédé selon la revendication 1 ou 2, caractérisé en ce que dans l'étape (a) en tant qu'ester de chloroformate de formule II on utilise un ester correspondant dans lequel $R^1$ est l'alkyle, de préférence le méthyle ou l'éthyle.

**4.** Procédé selon l'une des revendications 1 à 3, caractérisé en ce que l'on prépare un mélange d'un aldéhyde de formule IV avec un alcool de formule V dans lequel R est un groupe alcényle ayant 1 - 20 atomes de carbone ou un groupe alcényle substitué par un aryle.

**5.** Procédé selon l'une des revendications 1 à 4, caractérisé en ce que l'hydrure de bore métallique est utilisé dans l'étape réactionnelle (b) dans une quantité de 1,0 jusqu'à 1,5 moles par mole de l'acide carboxylique de la formule I utilisé à l'étape (a).

**6.** Procédé selon l'une des revendications 1 à 5, caractérisé en ce que la réaction de réduction (b) est effectuée à une température dans la plage de -50°C jusqu'à +30°C, de préférence dans la plage de -50°C jusqu'à 0°C.

**7.** Procédé selon l'une des revendications 4 à 6, dans lequel l'acide carboxylique de formule I est l'acide sorbique ou l'acide cinnamique.